Europäisches Patentamt

European Patent Office

Office européen des brevets

(11) Publication number: **0 290 443 B1**

(12) **EUROPEAN PATENT SPECIFICATION**

(45) Date of publication of patent specification: **02.03.94**

(51) Int. Cl.⁵: **A23L 3/26**, A61L 2/10, B65B 55/08, B65B 55/12, B65B 55/16

(21) Application number: **87900486.9**

(22) Date of filing: **05.12.86**

(86) International application number: **PCT/US86/02628**

(87) International publication number: **WO 88/03369 (19.05.88 88/11)**

(54) **METHODS AND APPARATUS FOR ASEPTIC PACKAGING OF FOODSTUFFS.**

(30) Priority: **13.11.86 US 930646**

(43) Date of publication of application:
**17.11.88 Bulletin 88/46**

(45) Publication of the grant of the patent:
**02.03.94 Bulletin 94/09**

(84) Designated Contracting States:
**AT BE CH DE FR GB IT LI LU NL SE**

(56) References cited:
DE-C- 817 898          FR-A- 2 195 408
FR-A- 2 452 968        GB-A- 521 189
GB-A- 1 432 095        GB-A- 2 076 620
US-A- 2 576 862        US-A- 2 635 051
US-A- 3 471 693        US-A- 3 922 349
US-A- 3 955 921        US-A- 4 112 124
US-A- 4 121 107        US-A- 4 175 140
US-A- 4 321 781        US-A- 4 348 357
US-A- 4 395 397        US-A- 4 396 582
US-A- 4 464 336

(73) Proprietor: **FOODCO CORPORATION**
**8888 Balboa Avenue**
**San Diego, CA 92123(US)**

(72) Inventor: **DUNNE, Joseph, Edward**
**3352 Del Rio Court**
**Rancho La Costa, CA 92008(US)**
Inventor: **CLARK, Reginald, Wayne**
**12919 Via Esperia**
**Del Mar, CA 92014(US)**
Inventor: **ASMUS, John, Frederich**
**8239 Sugarman Drive**
**La Jolla, CA 92037(US)**
Inventor: **PEARLMAN, Jay, S.**
**26 LaVista Verde**
**Rancho Palos Verde, CA 90274(US)**
Inventor: **BOYER, Keith**
**2511 35th Street**
**Las Alamos, NM 87544(US)**
Inventor: **PAINCHAUD, François**
**355 68e Rue Est**
**Charlesbourg, Ouebec G1H IR9(CA)**
Inventor: **HOFMANN, Gunter, A.**
**3750 Riviera Drive 6**
**San Diego, CA 92109(US)**

EP 0 290 443 B1

(74) Representative: **Cross, Rupert Edward Blount et al**
**BOULT, WADE & TENNANT**
**27 Furnival Street**
**London EC4A 1PO (GB)**

2

## Description

The present invention relates to methods and apparatus for the deactivation of organisms, microbes or viruses through the use of intense incoherent pulsed light.

Background of the Invention

Substantial technical effort has been directed to extend the storage time for foodstuffs and other microbiologically labile products and to preserve these products against microbiological spoilage. Such efforts have involved both the treatment of products and the development of packaging techniques for preservation.

Significant research and development effort has recently been directed to aseptic packaging technology for packaging of sterilized food products (including high and low acid foods) in sterile packaging materials, in order to provide preserved foodstuffs having an extended shelf life. However, such methods and apparatus may have various disadvantages such as requiring the extensive use of chemical disinfectants which may leave residual chemical products on the packaging material or foodstuff. New methods and apparatus for sterilizing food product packaging material and for aseptic packaging would be desirable.

The photobiological effects of light, including visible light (380-780 nm), near ultraviolet light (300-380 nm) and far ultraviolet light (190-300 nm), have been studied for many years, for example, as reported in Jagger, J., "Introduction to Research in Ultraviolet Photobiology", Prentice Hall, Inc., 1967, and efforts have been made to employ light to sterilize food products or containers for food products. US Patent No. 2,072,417 describes illuminating substances , e.g., milk, with active rays, such as UV rays. US Patent No. 3,817,703 describes sterilization of light-transmissive material using pulsed laser light. US Patent No. 3,941,670 describes a method of sterilizing materials, including foodstuffs, by exposing the material to laser illumination to inactivate microorganisms. US-A-4121107 describes an apparatus for automatically filling and packaging foodstuffs on an industrial scale, which employs a source of high intensity ultra violet radiation of a particular wavelength to disinfect the packaging material. The material is exposed to the high intensity light for at least one second and for up to 30 seconds to kill the most resistant microorganisms. However, such methods have various deficiencies, such as limited throughput capacity, limited effectiveness, adverse food effects, inefficient energy conversion (electrical to light) and economic disadvantages.

GB-A-2076620 describes a method of sterilizing deep layers of aspergillus niger using a flash discharge lamp containing a rare gas which emits intense rays of ultra violet radiation. The method is shown to be more effective in destroying the bottom layers of aspergillus niger than by employing a conventional sterilizer lamp.

It is an object of the present invention to provide a new method and apparatus for packaging of foodstuffs, particularly including improved aseptic packaging processes and apparatus.

These and other objects of the invention will become more apparent from the following detailed description and the accompanying drawings, of which:

FIGURE 1 is a schematic illustration of an embodiment of an aseptic packaging machine which continuously forms and fills a continuous packaging film and sterilizes the film by high intensity incoherent light pulses to provide aseptically packaged food products;

FIGURE 2 is a perspective view, partially broken away, of the high intensity incoherent pulsed light filling and sterilization assembly of the aseptic packaging apparatus of FIGURE 1;

FIGURE 3 is a schematic illustration of another embodiment of a packaging system which is adapted to aseptically package a sterilized food product in preformed containers which are sterilized by means of high intensity incoherent light pulses;

FIGURE 4 is a schematic side view of an embodiment of an aseptic packaging apparatus which forms and sterilizes containers from rolls of thermoplastic and lid materials;

FIGURES 5a and 5b are graphic representations of E. coli deactivation on culture media by means of high intensity incoherent light pulses;

FIGURES 6a and 6b are graphic representations of Bacillus subtilis (vegetative) deactivation on culture media by means of high intensity incoherent light pulses;

FIGURES 7a and 7b are graphic representations of Bacillus subtilis (spores) deactivation on culture media by means of high intensity incoherent light pulses;

FIGURES 8a and 8b are graphic representations of Stapnylococcus aureus deactivation on culture media by means of high intensity incoherent light pulses;

FIGURE 9 is a graphic representation of Saccaharomyces cerevisiae deactivation on culture media by means of high intensity incoherent light pulses;

FIGURES 10a and 10b are graphic representations of Aspergillus niger spore suspension deactivation on potato dextrose agar culture media supplemented with rose bengal, by means of high intensity incoherent light pulses;

FIGURE 11 is the spectral transmission curve of a glass filter adapted to remove far-UV from a pulsed flashlamp spectrum;

FIGURE 12 is a graphic representation of the nominal spectral output of the xenon linear flashlamps of a flashlamp system with the 5% and 50% transmission wavelengths of the glass filter of FIGURE 11 indicated by broken lines;

FIGURES 13 and 14 are graphic representations of E. coli deactivation using nonfat dry milk in the culture medium to increase its relative absorption of high intensity incoherent light pulses from which UV light has been filtered, and

FIGURE 15 is a graphic representation of E. coli deactivation using india ink in the culture medium to increase its relative absorption of high intensity incoherent light pulses from which UV light has been filtered.

## Summary of the Invention

The present invention is directed to methods and apparatus for the deactivation of living organisms, microbes or viruses on the surface of foodstuff packaging materials using intense, short pulses of incoherent, continuous, broad spectrum, polychromatic light.

According to the present invention there is provided a method for aseptic packaging of foodstuffs comprising the steps of providing an aseptic packaging material having a foodstuff contacting surface intended to form at least part of the interior surface of a sealed foodstuff container, applying to said foodstuff contacting surface a plurality of high intensity polychromatic, incoherent light pulses each having a duration in the range of from 0.001 to 100 milliseconds and an energy density at said foodstuff contacting surface in the range of from 0.01 to 50 joules per square centimetre, the light pulses including ultra violet light and light of longer wavelengths including infra-red at sufficient intensity and short pulse duration to produce pulsed thermal treatment of only a superficial surface layer of said packaging material, all to provide a sterilized foodstuff contacting surface, providing a sterilized foodstuff to be packaged, introducing the sterilized foodstuff into contact with said sterilized foodstuff contacting surface while excluding microorganisms from contact with said foodstuff and said sterilized foodstuff contacting surface, and sealing the sterilized packaging material to provide a sealed aseptically packaged foodstuff product.

Also according to the present invention there is provided an apparatus for performing a method of aseptic packaging of foodstuffs comprising pulsed light illumination means for applying to a foodstuff contacting surface of an aseptic packaging material a plurality of high intensity polychromatic light pulses having a duration in the range of from 0.001 to 100 milliseconds, an energy density at the foodstuff contacting surface in the range of from 0.01 to 50 joules per square centimetre to produce pulsed thermal treatment of only a superficial surface layer of the packaging material, means for conveying an aseptic packaging material to the pulsed light illumination means, sterilization means for providing a sterilized foodstuff to be packaged, means for introducing the sterilized foodstuff from the sterilization means into the sterilized packaging material surface provided by operation of the conveying means and the pulsed light illumination means while excluding microorganisms from contact with the foodstuff or the sterilized foodstuff contacting surface, and means for sealing the sterilized packaging material to provide a sealed foodstuff container.

Application of intense pulses of high intensity, incoherent polychromatic light provides efficient, effective, high throughput processing, including many practical and economic advantages. Moreover, the short duration of each pulse also permits under certain conditions, spatial localization of various of the lethal effects of the light pulses to a thin surface layer of the packaging material.

In one embodiment, the surface of the food packaging material is exposed to a plurality of pulses of incoherent light having a wavelength distribution such that at least 70%, and preferably at least 95% of its electromagnetic energy is distributed in a wavelength range of from 170 nanometers to 2600 nanometers, and a duration in the range of from $1 \times 10^{-6}$ to $1 \times 10^{-1}$ seconds, but preferably less than 10 milliseconds. Desirably, at least 40 percent, and typically greater than 70 percent of the energy of the light pulses should be of continuous emission spectra. Such short, intense, incoherent light pulses may be provided by pulsed, gas-filled flashlamps, spark-gap discharge apparatus, or other pulsed incoherent light sources. Pulsed, gas-filled flashlamps produce broadband light when an electrical current pulse is discharged through the flashlamp, ionizing the gas and producing an intense burst of both continuum and line emission over a broad spectral range. Such flashlamps typically employ inert gases such as Xenon or Krypton because of their high efficiencies of electrical to optical energy conversion. The use of other gases or gas mixtures and gas discharge systems is possible and may be desirable for specific applications. The application of an intense pulse of broadband light in accordance with various aspects of the present invention is believed to provide different lethal effects over a range of wavelengths at which the product to be treated absorbs light energy, in contrast to the effect of single line emission spectrum of, for example, a low power germicidal lamp.

Also in some embodiments of the invention, particular spectral distributions of the pulsed, high intensity incoherent light may be selected for particular purposes, by selection of the operating characteristics of the pulsed light source and/or by appropriate filtering. In this regard, for example, it is desirable for certain aseptic packaging processes that the packaging material be treated with pulses having a relatively high ultraviolet content, to minimize the total fluence necessary to achieve elimination of, or a predetermined reduction in, microorganism colony forming units (CFU). Far and near UV components of an incoherent, high intensity light pulse may be used for efficient and economic deactivation of microorganisms, microbes or viruses through photochemical effects so as to render them reproductively inactive on the surface of or within the near surface region of solid foodstuff packaging material surface, as will be described in more detail hereinafter. Spectral distributions and light pulse intensities which utilize a photothermal mechanism, such as through photothermal chromophores within the microorganism, microbe or virus, or through photothermal absorption of a surface or near a surface to be treated, are also contemplated herein. Both mechanisms may be utilized in highly efficient and effective treatment methods.

Desirably, the intensity of a particular wavelength distribution will be selected which will provide at least a reduction of initially present colony forming units at the surface to be treated by a factor of at least 10 (one log reduction, base 10) and more preferably at least one thousand (three logs reduction, base 10) upon treatment with at least two of the intense pulses of light. Reduction of colony forming units by a factor of at least a million or more (six logs reduction, base 10), ranging up to complete sterilization may be provided. The desired intensity will be less for UV-rich light pulses, and will be higher for processes which utilize a significant degree of surface heating for organism inactivation.

Aseptic packaging materials, may be subjected to intense, short pulses of UV-rich polychromatic incoherent light, which may or may not utilize surface heating to effect microorganism inactivation. In accordance with such methods, at least about 15 percent, and preferably at least about 30 percent of the energy of the light pulses will be at wavelengths shorter than 300 nanometers, and at least about 5 percent and preferably at least about 10 percent of the light pulse energy may desirably be at wavelengths shorter than 250 nanometers. Such UV rich pulses may typically have relatively low total energy density, such as in the range of from 0.01 to 15 joules per square centimeter, and typically from .1 to 3 joules per square centimeter. A single pulse of such UV rich light having a broad spectral range may produce effective sterilization of a desired substrate, and may be absorbed by and damage with lethal effect a broad range of different chromophoric groups of microbiological cell constituents, over a broad spectral range.

In addition to flashlamps, other pulsed light discharge devices producing appropriate broadband spectra and intensities may be used for the processes described herein.

Typically, packaging substrates may be exposed to between 1 and 20 pulses of high intensity, short duration incoherent light, with the use of a plurality of at least two pulses being particularly desirable. In various embodiments, the foodstuffs may be contained in a packaging material which is sufficiently transparent to the desired treatment spectrum prior to exposing its surfaces to the light pulses. In this regard, the packaging material containing the foodstuff to be treated may best transmit at least about 10% and more preferably at least about 50% of the energy of the light pulse over a predetermined treatment wavelength range less than about 320 nm. through the packaging material.

In accordance with various aspects of the present invention, particularly in respect to such methods in which the ultraviolet component of the pulsed light flashes is suppressed or substantially eliminated, the intensity of the pulsed light should be sufficient to heat a superficial layer of the packaging material having a thickness of less than $10^{-5}$ m (10 microns), at least 50° C. to a temperature of at least 75° C. and preferably at least 100° C. Such a very thin layer may be very briefly heated to a substantially higher temperature (e.g., greater than 150° C., such as in the range or 300° C. to 700° C.) concomitantly with the application of one or more light pulses. In this manner, heat may be localized at a very superficial surface layer to kill surface microorganisms without significantly raising interior temperatures of the material. For purposes of the invention, microorganisms are considered to be inactivated if they are either killed or rendered reproductively inactive. During the interval between successive pulses, the heat which is deposited in the surface layer of the packaging material may be conducted and dissipated interiorly without significantly altering the product. The number of light pulses and their total energy may be limited so as to maintain the measurable surface temperature of the material, ten seconds after pulsed light exposure, below about 100° C., and preferably to limit the surface temperature increase resulting from pulsed light treatment, at least 10 seconds after such treatment, to less than 50° C., and more preferably, less than 15° C.

In some embodiments, treated material may be substantially opaque to the light to which it is exposed so that very little light penetrates into the material and substantially all of the light (other than that which is reflected) is dissipated within a very superficial surface layer typically between less than about 1 micrometer and up to 1 millimeter thick. Light penetrates into a material surface according to an exponential formula:

$$I = (1-R) I_o e^{-\alpha x} \qquad (1)$$

where I is the energy intensity of the light transmitted to a distance below the surface, R is the surface coefficient of reflection, $I_o$ is the intensity incident upon the surface, and $\alpha$ is the extinction coefficient which measures the opacity to light of the material being used. The light which penetrates the material but is not transmitted is dissipated as heat in the material. At any point into the surface, the energy per unit are ($E_d$) dissipated in a depth, d, is given by the formula:

$$E_d = (1-R)I_o [1-e^{-\alpha d}] \qquad (2)$$

As soon as the heat is deposited in the material through absorption of the light pulse, it begins to spread by thermal conduction generally in accordance with the well known law of heat transfer:

$$E_c = Akt \frac{dT}{dx} \qquad (3)$$

where $E_c$ is the energy in the material which is conducted between two planes of area A separated by a unit of length dx, k is the thermal conductivity of the medium, dT is the difference of temperature between the two same planes in degrees Kelvin, and t is the time is seconds allowed for the heat conduction process to take place. In some embodiments, the treated material will have, or will be pretreated by means of an appropriate absorption enhancing agent to have an appropriate effective average absorption extinction coefficient ($\alpha$) over the desired spectral band of wavelengths to provide the desired absorption of energy within an appropriate depth.

When a beam of continuous light is absorbed at a material surface, it is transformed into heat in the material generally according to equation (2); the heated surface becomes hotter, establishing a temperature gradient in the material and leading to a flow of heat into the deeper layers of the material at a rate set generally in accordance with equation (3). Eventually, a steady-state is established where the surface temperature is such that as much heat flows into the depth of the material as is deposited in the surface by the light beam.

In accordance with certain aspects of the present invention, heating of a superficial layer of a relatively opaque packaging material is effected with light sources capable of producing pulses that each supply energy densities of between 0.01 and 50 joules per $cm^2$ and preferably between 1 and 20 joules per $cm^2$ to the surface of the material during the duration of the pulse. For example, light pulses having an energy content between 2 and 20 joules per $cm^2$ (e.g., between 8 and 16 joules per $cm^2$) may readily and effectively be applied to the surface. Typically, the energy density of the light pulses applied to the surface is sufficient to produce pulsed thermal treatment of a very superficial surface layer. In order that the surface temperature is elevated before significant amounts are conducted interiorly, this energy is desirably supplied in pulses having a duration in the range of from 0.001 to 100 milliseconds, and preferably from 0.1 to 3 milliseconds, such as between 0.1 and 1 millisecond. The duration of a pulse is determined by the elapsed time between when the rising light energy density of the light pulse is half of its peak value and when the intensity has fallen to half of its peak value. The total amount of light energy that will be supplied to each type of product depends upon properties of the particular material, such as its extinction (or absorption) coefficient and its surface coefficient of reflection. For methods utilizing surface heating, the requisite amount of heating for the particular packaging material also depends to a limited extent on the type or types of surface organisms, microbes or viruses which must be destroyed.

For treatment processes in which it may be desired to limit the application of UV light to the product, the supplied light may be distributed primarily in wavelengths that range through the visible and into the far and near UV and near IR and preferably at least about 80% of the energy of the light pulse is distributed in the wavelength range between 270 and 2600 nanometers. For example, in certain specific treatment materials, the supplied light may be distributed primarily in wavelengths such that at least about 90% of the energy of the light is distributed in the wavelength between 300 and 2500 nanometers. Such light pulses may have at least 10% of their light energy distributed in the near UV wavelengths, i.e., between 300 and 400 nanometers. However, visible and infrared light are also very effective in producing a desired thermal surface effect. If desired, part or substantially all of the light at a predetermined cut-off frequency or a particular bandwidth may be eliminated, as by filtering, from the pulsed light spectrum. Such filtering may be accomplished by means of solid filters such as UV absorbing glass filters, or by liquid filters such as provided by a static or flowing liquid jacket surrounding a flashlamp having undesired spectral components. The liquid jacket may contain appropriate organic or inorganic absorption agents, such as inorganic salts which absorb at wavelengths which are to be removed. For example, a solution of copper sulfate in water used as a flashlamp cooling jacket medium (e.g., 50 grams $CuSO_4$ per gallon of water) provide an effective UV filter in the far UV. The absorption spectra of solid filter materials, liquids and solutions of organic and inorganic materials are well known, and may be selected as desired.

It is found that short, high intensity far and near UV pulses can very effectively deactivate vegetative and spore forms of microorganisms by thermal and/or photochemical means. The use of short, intense light pulses is found to allow a significant reduction in product processing time and significantly increase product throughput. However, pulsed visible and infrared light are also effective in producing the desired effect in highly absorptive media through surface heating. The ability to inactivate organisms, microbes or viruses on surfaces with broad spectrum light makes it possible to more effectively inactivate microorganisms (e.g., microbes or viruses) by applying the incoherent, broad spectrum light pulses through transparent packaging materials, such as glass or clear plastic, some of which may tend to absorb certain ultraviolet wavelengths.

Much of the heat that is produced in the surface will eventually be conducted into the interior of the product; however, the total quantity of heat that is produced, even by a series of pulses, may be small relative to the amount of heat that would be needed to substantially raise the temperature in the interior of the product. Under these circumstances, the product (except for a very superficial surface layer) is not heated to a temperature that would substantially alter its characteristics. Moreover, the number of pulses used to reduce the microbiological burden on the surface of a product is desirably limited so as not to overheat the product.

A plurality of the closely spaced pulses of intense light, and in some cases a single pulse, will substantially reduce the population of microorganisms, typically by greater than about one order of magnitude (base 10) and preferably at least two orders of magnitude. Higher levels of reduction (including complete sterilization) may be accomplished at appropriate energy levels and treatment pulse numbers. Usually between 1 and 50 pulses of light are used to sufficiently treat a packaging material surface and preferably between 1 and 20 pulses are used. It is highly desirable that a plurality of at least 2 of the high intensity light pulses be applied.

In some embodiments high energy light pulses which contain a substantial proportion of ultraviolet radiation may desirably be employed. If the surface that is being sterilized is of a food product container, the surface will commonly be subjected to between 1 and 20 pulses of light, typically from 1 to 3 pulses, to assure adequate microorganism inactivation. However, higher numbers of pulses, such as between 5 and 20 pulses, may be used with lower power levels, and/or to obtain increased deactivation.

The interval between pulses of high intensity light which are applied to a packaging material should be long enough for some of the heat to dissipate from the superficial surface layer, yet short enough so that the multiple pulses have cumulative effect. The time between pulses applied to the surface being treated desirably may be generally between 0.001 seconds and 30 seconds (e.g., 0.1 to 5 seconds), and preferably less than about 2 seconds in commercial packaging applications. When the pulses are provided by a single flashlamp (or flashlamp assembly of a plurality of lamps which are flashed simultaneously), the maximum repetition rate is governed as a practical matter by individual lamp cooling parameters, which will typically provide a repetition rate in the range of from less than 1 to 1000 times per second. However, the effective repetition rate may be increased by employing multiple flashlamps which are sequentially flashed, and by providing relative movement between the flashlamp and the surface being treated.

Incoherent pulsed light of sufficient intensity as well as appropriate duration and wavelength distribution is obtainable from a flashlamp system. A suitable flashlamp system is sold by Maxwell Laboratories, Inc., under the trademark Flashblast. A particular model, the Flashblast Model FB-300, consists of a DC power supply which charges energy storage capacitors, a switch used to control the discharge of these capacitors, a trigger circuit to fire the switch at pre-programmed time intervals (automatic mode) or when a button is depressed by the operator (manual mode), a set of high voltage coaxial cables carrying the discharge pulses from the capacitor-switch assembly, and from one to four flashlamps mounted in metal reflectors to direct the light emitted from the lamps.

In order to enhance the effect of high intensity, pulsed incoherent light treatment, particularly for transparent, reflective or relatively nonabsorbent substrates, a suitable absorption enhancing agent may be applied to the surface of the packaging substrate.

In accordance with various aspects of such methods, an absorption enhancing agent may first be applied to the surface to be treated. The agent may be applied in any suitable manner, such as by spraying or dusting the surface with a powder containing the agent or by applying the agent as a dissolved liquid, such as an aqueous or nonaqueous solution of the agent which may be applied by spraying, coating or immersing the substrate to be treated, or by vaporizing the agent onto the surface of the packaging material.

Suitable absorption enhancing agents should have a high optical absorption coefficient at the spectral wavelengths desired, within the spectral range of the high intensity light pulse(s) used in the treatment. Although the agent may be substantially completely removed from the product by the processing, the agent should best be an edible material which is generally recognized as safe and which may be readily applied to packaging surfaces which are to contact the surface of the food product.

Desirably, the agent may be selectively absorbed onto living cell surfaces, so that the amount of agent used may be minimized or its effect concentrated. Indicator agents such as dyes which are photon sensitive, pH sensitive or which are sensitive to oxidation potential may be utilized to processing advantage, so that the photonic absorption of the agent may be varied as part of the treatment process. Such indicator dyes may be useful for particular packaging films or treatment procedures in which the dye absorption is increased or decreased during pulsed light treatment. Absorption enhancing agents may desirably be selected which vaporize without decomposition or which have benign decomposition products. Examples of agents include approved Food, Drug and Cosmetic colors such as carotene, red dye #3, lime green, black cherry and mixtures thereof. The various natural dyes and natural food colorings may desirably be used for food product processing as may various natural or cooking oils. Mixtures of two or more components having different absorption maxima may desirably be used to increase optical absorption over the desired spectrum.

After application of the absorption enhancing agent to the product surface from solution (such as by dipping, spraying or roll coating), excess solution may be removed, and the surface of the product may be partially or completely dried if desired. The product may subsequently be subjected to pulsed incoherent light treatment, to heat a very thin surface layer, which has been subjected to agent treatment, in a time which is small compared to the time required for thermal conduction.

The use of absorption enhancing agents for pretreatment of products may allow the pulse width of the light provided by the flash apparatus to be increased. This has the effect of lowering the ultraviolet content, shifting the output of the flashlamps to longer wavelengths and increasing the service life of the flashlamps.

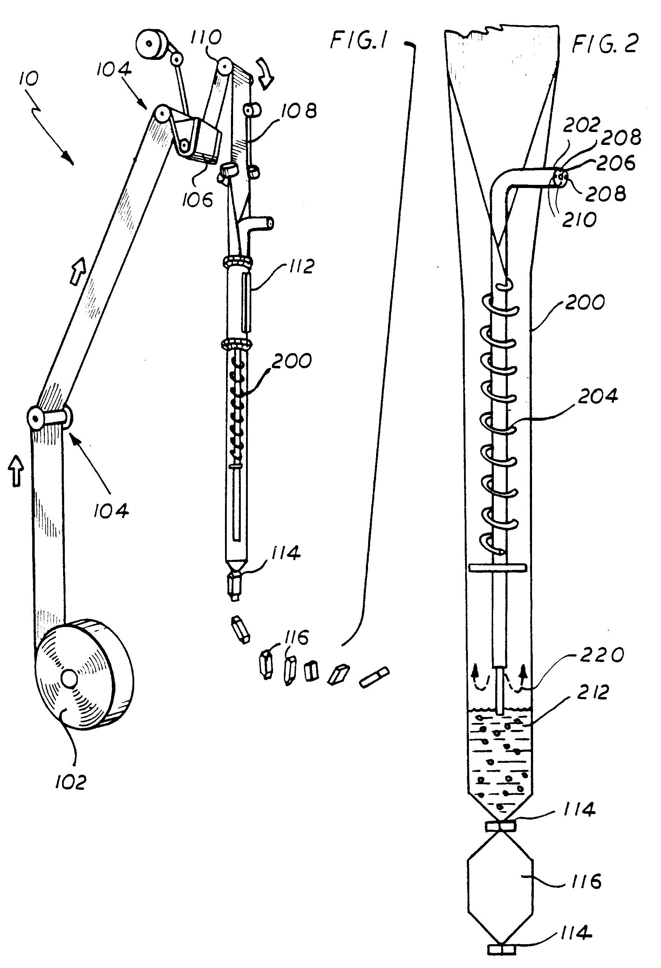

Various aspects of the invention will now be more fully described with respect to the specific embodiments illustrated in the FIGURES and various Examples. In this regard, illustrated in FIGURE 1, is an aseptic packaging apparatus 10 in which a reel of conventional flexible aseptic packaging material 102 is directed by means of a series of rollers 104 in accordance with conventional practice, to a solution of an optional absorption enhancing agent as previously described, in dipping-trough 106. The packaging material may typically comprise a layered structure of one or more internal coating and sealing layers, a metal foil such as aluminum foil, a laminating layer or paper layer and an external layer, in accordance with conventional practice.

Excess absorption enhancing agent solution may be removed by rollers 110, with the film being subsequently formed into a longitudinally sealed tube by longitudinal sealing apparatus 112. Depending upon whether a lap seal or a fin seal is desired, a strip 108 may be applied to one edge of the packaging material to reinforce the longitudinal seam, and to prevent the product from coming into contact with the edge of the film 102.

An important aspect of aseptic packaging apparatus 10 is product filling and flashlamp assembly 200 which is shown in more detail in FIGURE 2. The illustrated assembly 200 comprises an outer support tube 202, having attached thereto one or more flashlamps 204 distributed about and along the tube 202 such that upon pulsing, the entire inner surface of the sealed packaging material tube is subject to intense, short duration incoherent light pulses. A variety of arrangements of the flashlamps along the support tube 202 is feasible, the essential feature being that the entire inner surface of the packaging material tube is exposed to the pulsed light. Internally of the support tube 202 is a sterile food product tube 206, and flashlamp electrical cable 208 and optional lamp coolant lines 210 may be located intermediate the tubes 202, 206. In addition, sterile air provided under pressure from a suitable supply (not shown) may be conducted for discharge within the sealed tube. Sterile air may be produced by a variety of techniques (filtration, incinernation) including the use of intense incoherent light pulses as described herein. In operation, the longitudinally sealed film tube, which is transversely sealed by a suitable transverse sealing apparatus 114 has introduced therein a predetermined portion of substantially sterile food product 212. The sterilized food product may be produced by short time, high temperature processing or by other processes. The longitudinally sealed film tube is advanced one package length, while the flashlamp assembly is pulsed a plurality of times in order to repeatedly sterilize the entire adjacent interior of the tube above the food product 212. Sterile air 220 exits the support tube 202 and is carried over the flashlamp assemblies to cool the flashlamps and to remove from the longitudinally sealed film tube any ablation products produced by the flashlamp discharge and to prevent contamination from settling on the treated area. Following transverse sealing, the packages may be separated into individual consumer packages 116.

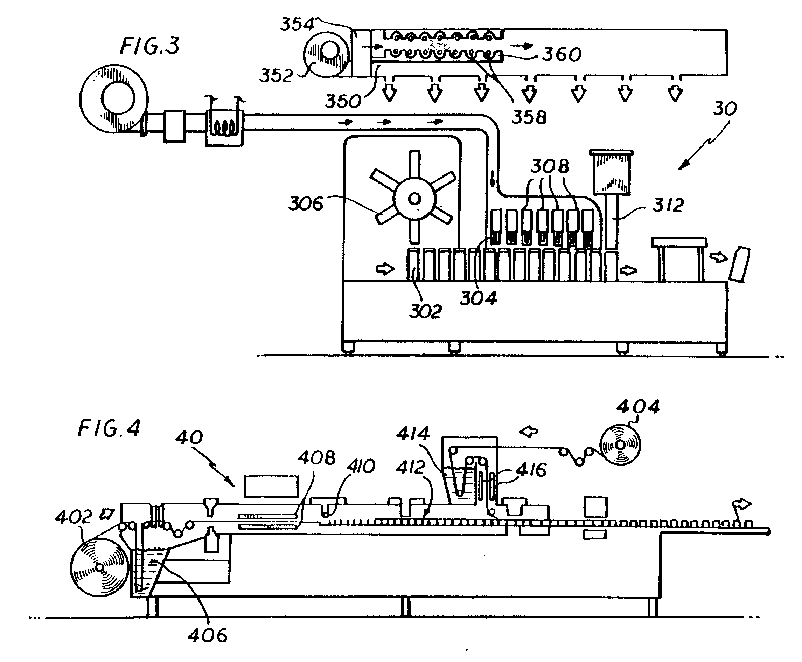

The present method may also be applied to other types of aseptic packaging systems, such as those which utilize preformed product containers. In this regard, illustrated in FIGURE 3 is aseptic packaging apparatus 30. The packaging apparatus 30 utilizes preformed product containers 302 which are introduced into the sterilization zone 304 of the apparatus 30. Optionally an absorption enhancing agent solution as previously described may be sprayed into containers 302 by means of spraying apparatus 306. Subsequently, the containers progressively pass through a plurality of flashlamp treatment stations 308 in which reciprocating "U" shaped flashlamps, linear flashlamps, bulb type flashlamps and/or flashlamps of other configurations are introduced above or into the container openings and the flashlamps pulsed at least once per container 302. The treatment stations are then withdrawn and the containers are advanced by one station, as the process is repeated so that the entire interior surface of each of the containers is subjected to a plurality of intense incoherent light pulses as it progresses along the treatment stations. A sterile air purge apparatus may also be utilized to remove any material ablated from the interior of the containers and to prevent contamination from settling in the treated containers and to cool the flashlamps. A suitable stationary battery of flashlamps may also be provided to treat the exterior and edge surfaces of the containers upon their passage through the flashlamp treatment zone if desired. The sterilized containers subsequently pass through the filling station 312 where a preprocessed food product is introduced into the container, which is subsequently sealed at the top by a sterile lid.

A laminar, sterile air curtain may be provided over the entire aseptic packaging apparatus 30 in order to prevent the infection of the packaged units. The sterile air may be provided by gas sterilization apparatus 350 which includes an air input blower 352, which pumps air through filter 354 to a pulsed light treatment zone 356 containing a bank of high power Xenon flashlamps 358 enclosed in a reflective housing 360. The air is continuously forced through the zone 356 at a rate which in conjunction with the pulse rate of the lamps 358, insures that all of the air is subjected to a plurality of high intensity polychromatic incoherent light pulses as previously described, as it passes through the zone 356. Desirably, the light pulses will be a UV rich (e.g., having at least 15 percent of the light energy at wavelengths shorter than 300 nanometers) and will desirably have an energy density of at least 0.5 joule per square centimeter throughout the treatment zone through which all of the air passes. The pulse duration may typically be in the range of from about 0.1 to 3 milliseconds. The multiple-lamp reflector array provides multidirectional, substantially even illumination to the air or other gas flowing therethrough, so that a dust particle or bacterial colony forming unit is treated from all sides and is not self-shielded. This multidirectional treatment is an important feature of the system 350.

Illustrated in FIGURE 4 is an additional embodiment of an aseptic packaging apparatus 40 which comprises two reels 402, 404 of plastic packaging material, one for the body of the finished packages and one for package lids. The container body material may be conducted through an optional absorption enhancing agent bath 406 as previously described. The packaging material 402 may be conducted through a suction and drier section to remove excess agent solution. The packaging material is subsequently subjected to intense incoherent light pulses by an array 408 of flashlamps extended longitudinally along the direction of travel of the packaging material, following which the packaging material 402 may be thermoformed into suitable containers and forming apparatus 410 which are then filled with an aseptically processed foodstuff at filling station 412. The lid material may similarly optionally be passed through an absorption enhancing agent bath 414, subjected to a plurality of intense incoherent light pulses by flashlamp array 416 and utilized to seal the filled formed containers. The entire apparatus may be maintained under a sterile air blanket.

In the sterilization of packaging materials the full broad spectrum flashlamp output including near and far ultra violet light components of the spectrum will normally be employed, so that relatively low fluences may be utilized. For example, even at very high organism densities (up to $1 \times 10^6$ to $1 \times 10^8$ CFU/cm$^2$), only one or two flashes at an energy density of 1.5 J/cm$^2$ per flash will result in sterilization of both spores and vegetative bacteria and viruses.

When, as is preferred, the flashlamp spectra includes at least about 10 percent of its energy at wavelengths shorter than about 300 nanometers or when the product itself is sufficiently absorptive to provide the desired spectral interaction, the absorption enhancing agent bath of the aseptic packaging apparatus, as previously described may desirably be eliminated. For high speed operation and high power densities, it may be desirable to cool the flashlamps by optional water jacket. For transparent wrapping or other packaging materials, it may also be desirable to provide a lamp array externally of the longitudinally sealed film, so that only the product and countercurrent sterile air tube is inserted in the film tube.

Various aspects of the invention will now be described in greater detail by way of the following specific examples. These examples demonstrate the effectiveness of certain embodiments for reducing or eliminating microorganisms from bacteriological media and packaging materials. In some examples, microorganisms were deliberately introduced onto the surfaces to be treated and in other examples the materials were treated to remove the naturally occurring microorganisms. It is noted that the deliberate application of very high densities of microorganisms to surfaces produces a high degree of self-shielding of the microorganisms. This high degree of self-shielding increases the intensity and/or number of pulses of light required to achieve a given ratio of reduction of colony forming units over that required for the same reduction ratio for a lower density of indigenous colony forming units. Accordingly, these examples using high microorganism density represent a dramatic demonstration of the effectiveness of the pulsed light treatment.

EXAMPLE 1

In order to demonstrate the effectiveness of intense, pulsed incoherent light flashes in aseptic packaging and food preservation uses, a series of tests were carried out in which cultures of various microorganisms, which are representative of naturally occurring food spoilage microorganisms, were inoculated onto the surface of a culture medium. The inoculated culture medium subsequently was subjected to intense, incoherent pulsed light under a variety of test conditions. The light pulses were provided by the FB-300 Flashblast pulsed light generation system of Maxwell Laboratories, Inc. The FB-300 Flashblast pulsed light system has a linear Xenon flashlamp in a reflective housing through which is discharged, under control of a high current switch, a 745 microfarad capacitor bank which may be charged to 2600 volts to produce a maximum energy of 2500 Joules. The Xenon flashlamp has a highly UV transmissive fused quartz envelope with a 7 millimeter bore, an arc length of about 22.5cm (9 inches) and was filled with Xenon at a pressure of $6 \times 10^4 \, Nm^{-2}$ (450 Torr).

The spatial photolamp reflector housing of the pulsed light system used in the following examples was designed for no more than a 25% fluence variation over a sample test area of 2.5 cm by 10 cm (1 inch by 4 inches).

Stock Aspergillus niger and Saccharomyces cerevisiae cultures were grown on Potato Dextrose Agar (PDA) (pH 5.6, not further acidified with tartaric acid) at 25° C. Aspergillus niger mold spore suspensions were collected in 0.1% sterile Tergitol 7 (anionic) and treatment platings carried out on PDA containing 0.05% Rose Bengal (acid red 94: tetraiodotetracholorofluorescein, Na Salt) to inhibit mycelial spreading. Saccharomyces cerevisiae and all other microbial cultures except Aspergrillus niger were treated on Tryptic Soy Agar (DIFCO). Bacillus subtilis vegetative cultures were grown in 15 ml of Tryptic Soy Broth as an unshaken flat culture in a 10 x 100 mm petri at 35° C. Other bacterial cultures were grown as 10 ml deep at 35° C. The Bacillus subtilis spore suspension was a purified spore population containing greater than 95% spores and was stored in distilled water at 4° C. and diluted for plating immediately prior.to treatment. Fungal cultures were incubated at 25° C. while bacterial cultures were incubated at 35° C.

Stock cultures, the Bacillus subtilis spore suspension and Aspergillus niger spore suspension were diluted serially prior to treatment in sterile 1/10th strength tryptic soy broth. Twenty-five microliter droplets of the undiluted stock suspension and $1 \times 10^{-1}$ through $1 \times 10^{-6}$ dilutions of the stock suspension were spotted in two rows along the central chord of a petri dish containing approximately 15 ml of tryptic soy agar. The approximate width of the two row spot pattern was slightly less than 2.5 cm (one inch). The droplets were air dried at 35° C. and then exposed to intense, incoherent pulsed light.

After exposure and incubation of plates, the growth patterns of control and exposed plates were examined. The growth pattern on treated plates was recorded for comparison and for determining the degree of inactivation in factors of powers of 10 (each power of ten reduction being referred to as one "log"). Treatments yielding no survivors were recorded as X logs, where the " " sign indicates no survivors, and where X indicates the analytical limits of the testing.

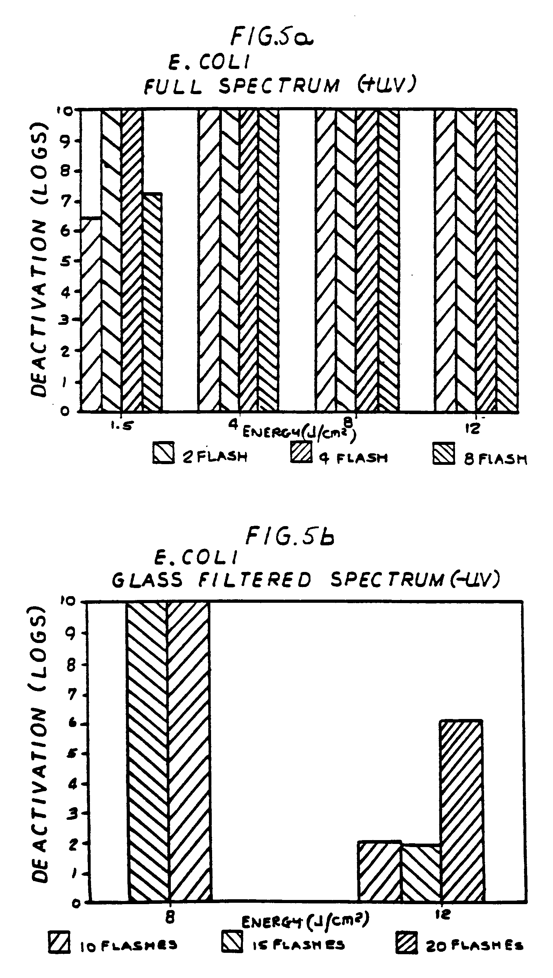

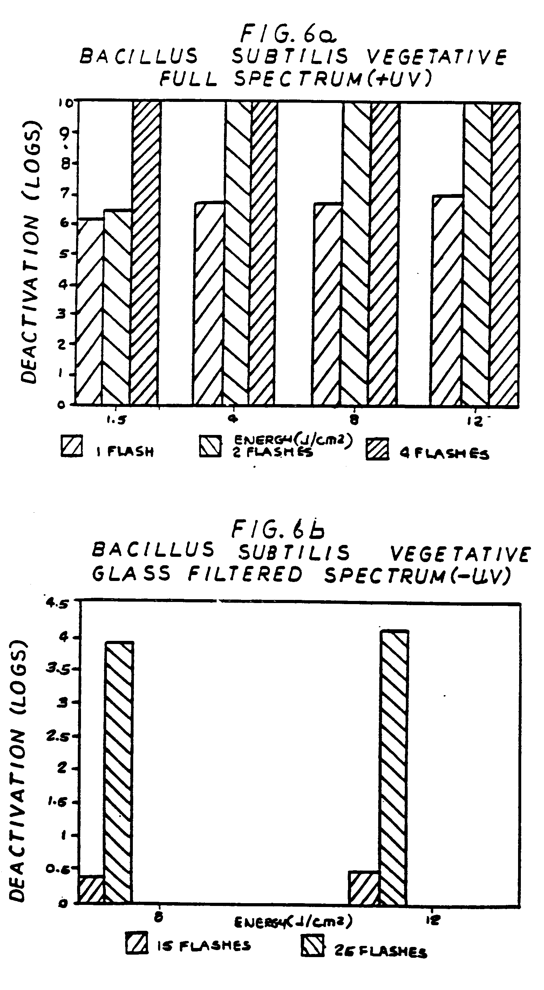

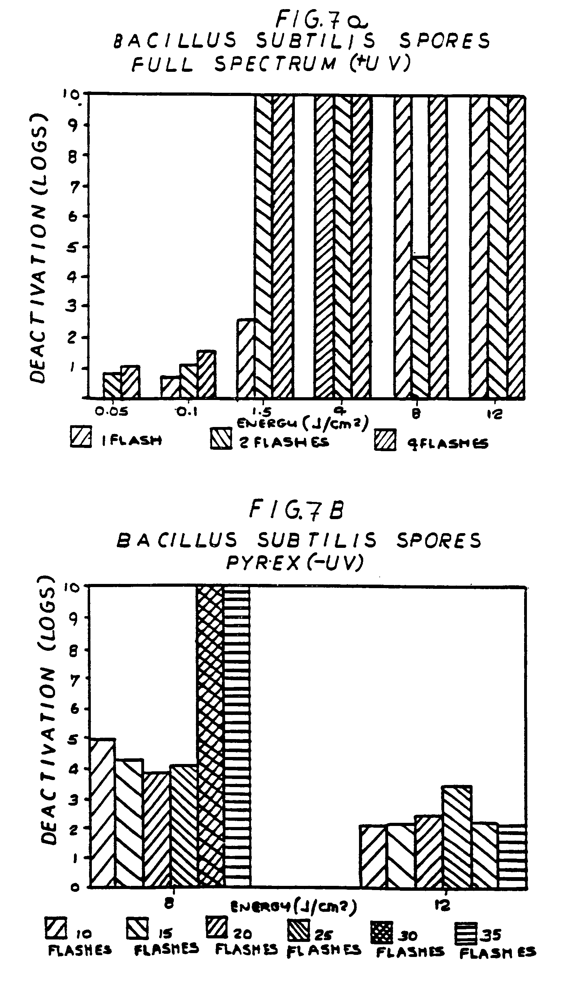

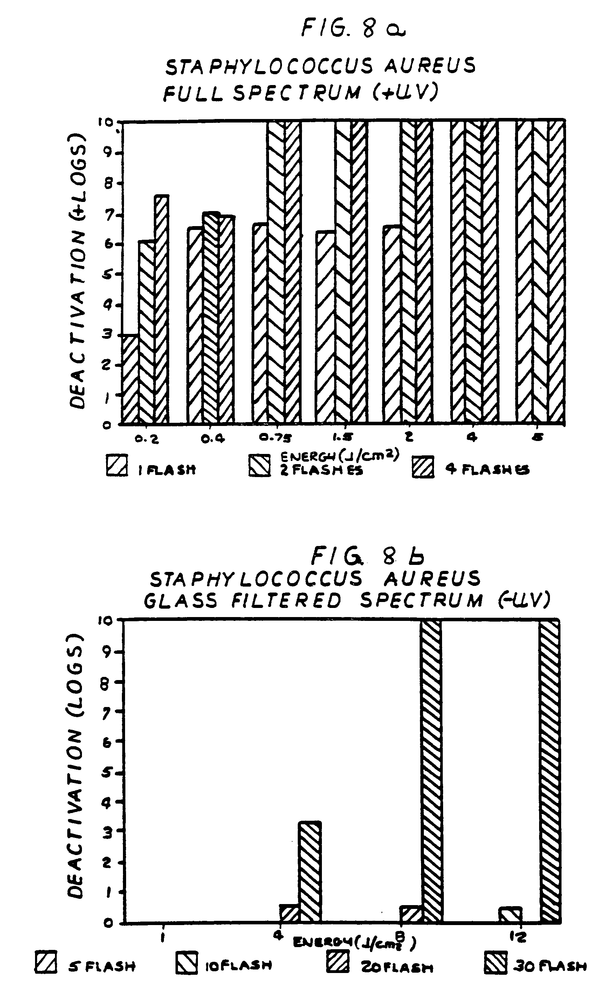

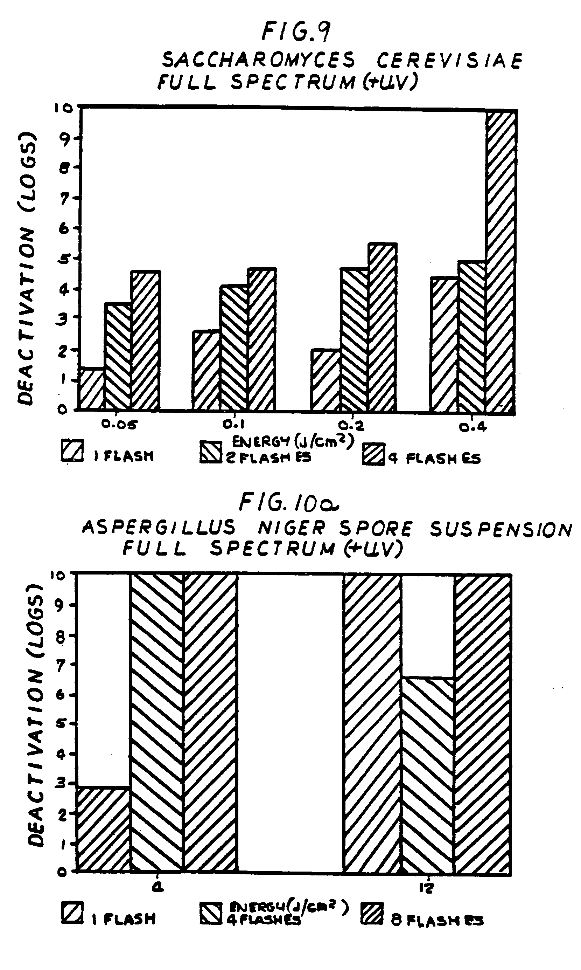

The results of the deactivation tests are given in FIGURES 5 through 10. Generally, deactivation using an unfiltered spectrum was substantial, with complete sterilization being observed in many cases. Even at very low fluences of 0.1 J/cm$^2$ or less, several logs of deactivation were observed. When the far-UV

10

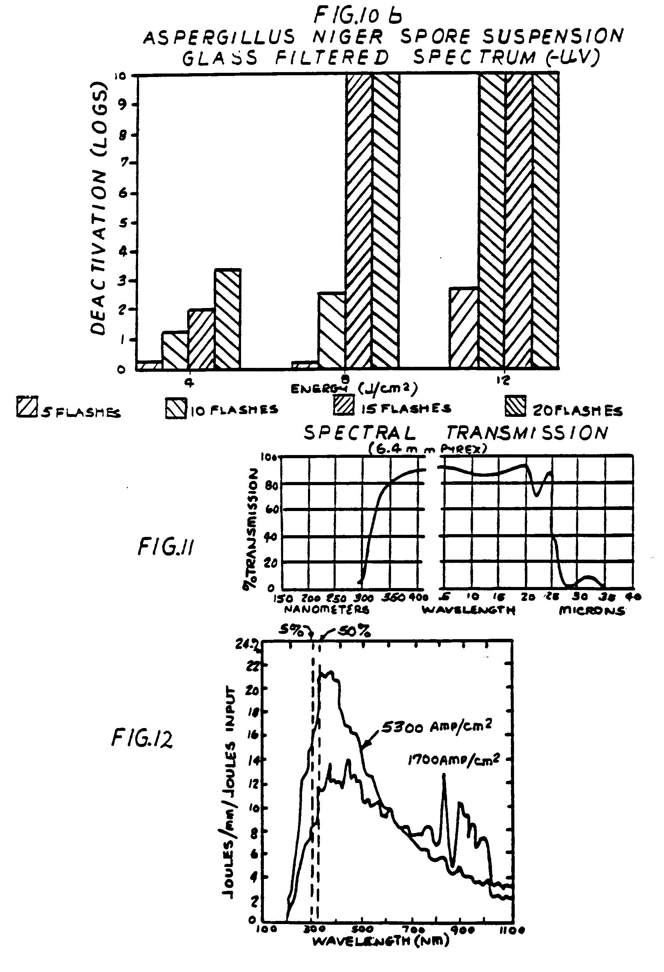

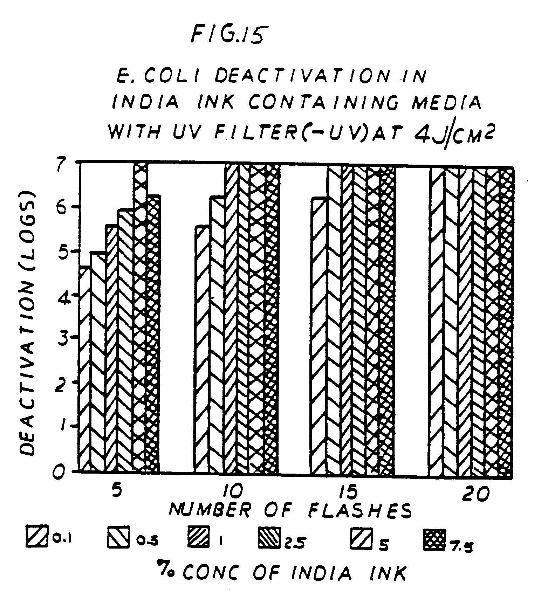

illumination was substantially eliminated from the pulsed light spectrum through the use of a 6.4 mm (1/4 inch) thick glass (Pyrex) filter, the fluence and number of flashes utilized to produce a similar deactivation effect increased substantially. The spectral transmission curve of glass UV filter is shown in FIGURE 11. The nominal spectral output of a Xenon linear flashlamp of the FB-300 system is shown, together with the 5 percent and 50 percent transmission wavelengths (respectively shown by dotted line) of the glass filter of FIGURE 11, providing a steep transmission cutoff at about 300 nm for pulses transmitted through the glass filter. Generally at fluences of 8 to 12 J/cm$^2$, ten flashes were employed to produce greater than one log of deactivation. At these energy levels, sterilization was obtained after a larger number of flashes (from 15 to 30 depending upon the sample). Moreover, a threshold effect is apparent from the test data of FIGURES 5-15 in which a slight increase (less than 20%) in treatment intensity produced a dramatic increase in response deactivation.

EXAMPLE 2

As previously indicated, the provision of incoherent light pulses having significant far UV content substantially reduces the fluence of the light pulses which are necessary for a predetermined degree of lethal effect for treatment of a package material substrate. In the absence of significant far UV content in the light pulses, absorption of the substrate (and/or microorganisms) in the near UV, visible, and near infrared wavelength range is important to provide high microorganism deactivation.

For example, in tests like those of Example 1 in which E. coli was treated with and without a glass filter for 1, 2 and 4 flashes at 1.5, 4.1, 8 and 12 J/cm$^2$, no survivors were observed on any plates treated with the full spectrum, and no effect was observed with the filter in place at these treatment levels.

It was noted, however, that the bacteriological medium used was substantially transparent to the intense incoherent illumination. Thus a 5 mm thick layer of tryptic soy agar contained in the plastic petri dishes used, attenuated the light by only about 35% and the majority of this attenuation occurred at wavelengths less than 300 nm which represented only about 15% of the total incident illumination. It was thus concluded that when the treatment spectrum was filtered by the use of the 1/4 inch pyrex glass filter to remove wavelengths less than 300 nm from the light incident on the sample, the absorption of the light at the surface of the bacteriological medium was insufficient to produce efficient coupling of the light energy to the treated surface.

To demonstrate the effect of media absorptivity (and surface characteristics) on pulsed light deactivation with the Pyrex ultraviolet light filter in place, an E. coli culture was seeded on a 45 mm diameter, 0.65 micrometer pore diameter white Millipore filter and upon similar filters which had been colored black by india ink.

Seeded filters were treated in a sterile petri dish and then laid bacteria-side up on the surface of a tryptic soy agar plate. Bacteria seeded on a white millipore filter exhibited greater than 6 logs deactivation after 4 flashes at 12 J/cm$^2$. This compares to substantially no deactivation observed using bacteria seeded directly on tryptic soy agar and exposed to 4 flashes at 12 J/cm$^2$. Similarly, bacteria on a black millipore filter required only one flash at 5 J/cm$^2$ to produce greater than 6 logs deactivation.

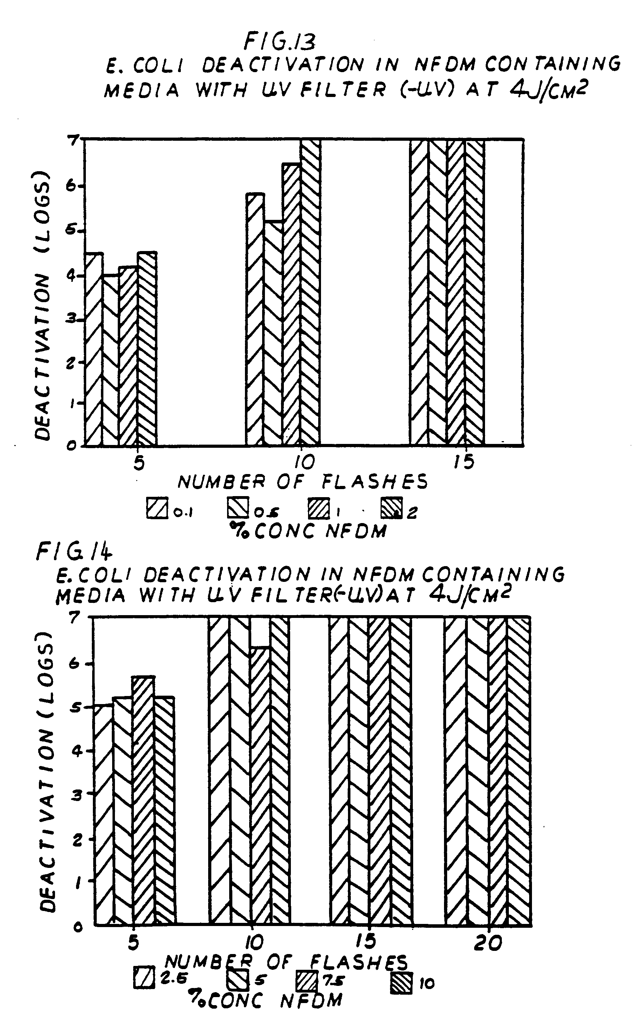

As a further demonstration of the effect of medium absorptivity on pulsed light deactivation with the 1/4 inch Pyrex glass UV filter in place, a series of experiments were performed in which nonfat dry milk or india ink were added to the bacteriological medium in order to increase its relative absorption of the intense, incoherent pulsed light. The results obtained are shown in FIGURES 13-15 which illustrates the deactivation of E. coli on media containing various concentrations of nonfat dry milk (NFDM) or india ink treated with 5, 10, 15 or 20 flashes of 4 J/cm$^2$ incident illumination with the Pyrex glass filter in place.

The mechanism for deactivation using pulsed light wavelengths greater than 300 nm is believed to be different from that observed when employing spectral output including a broad far-UV spectrum. Deactivation using the full flashlamp spectrum is believed to be similar to the effects of far-UV in dose and deactivation kinetics; deactivation using only the spectral output greater than 300 nm is believed to demonstrate a threshold relationship between dose and deactivation. This mechanism is believed to be produced by flash heating of the medium surface and varies with the ability of the uppermost layers of the medium to absorb (i.e., interact with) the incident spectral fluence during the short flash period.

Thus, two mechansisms using intense incoherent pulsed light are believed to effect microorganism inactivation, which are respectively photochemical and photothermal mechanisms. Both mechanisms may be present in effective treatment processes.

11

## EXAMPLE 3

A series of tests were performed to determine the effectiveness of intense incoherent pulsed light for the sterilization of packaging material. The lid and cup material used by a leading U.S. food processing company for packaging a dessert pudding product were chosen as sample substrates. The cup stock material was comprised of a polyethylene/ethyl vinyl alcohol oxygen barrier plastic/polyethylene laminate. The lid stock material was comprised of an aluminum/polyethylene laminate. Small pieces of each material were sterilized by autoclaving, hydrogen peroxide (3% or 10%) and heat, or UV-germicidal light; the subsequent results obtained were not affected by which pre-sterilization technique was used. One polyethylene surface of each of many samples of each material were then seeded uniformly with either Staphylococcus aureus in saline, Bacillus cereus spores in water, or Aspergillus niger spores in 0.1% Tergitol 7 and then air dried at 35° C. The seeding concentration was between 100 and 1000 CFU per square centimeter of surface area. Seeded pieces of lid and cup stock were then flashed with intense incoherent light using the FB-300 system. A single pulse of 1.5 millisecond (full width, half maximum) duration was given at the total light energy per square centimeter recorded. Then treated and control lid and cup stock samples were incubated individually in sterile tryptic soy broth at 35° C. for Staphylococcus and Bacillus seeded samples and room temperature (21° C.) for Aspergillus samples. The results obtained are recorded in Table 1 where a + (plus) indicates growth was detected and a - (minus) indicates no growth was detected after one week of incubation.

TABLE 1

| Deactivation of Vegetative and Spore Bacterial and Fungal Samples Using a Single Light Pulse | | | |
|---|---|---|---|
| Energy (Joules/cm) | Staph. Aureus | Bacillus Cereus Spores | Aspergillus Niger Spores |
| 3.0 | - | - | - |
| 2.75 | - | - | - |
| 2.50 | - | - | - |
| 2.25 | - | - | - |
| 2.0 | - | - | + |
| 1.75 | - | + | + |
| 1.5 | - | + | + |
| 1.25 | - | + | + |
| 1.0 | + | + | + |
| 0.75 | + | + | + |
| 0.5 | + | + | + |
| 0.25 | + | + | + |

These results demonstrate the ability of intense incoherent pulsed light to sterilize conventional polythylene laminate packaging materials.

This invention had wide application to the deactivation of organisms upon the surface of or within the near surface region of packaging materials.

The use of intense incoherent pulsed light in the treatment of food packaging materials for the deactivation of organisms is found to have many advantages when compared to the use of conventional continuously operating light sources or laser light sources operating in a continuous or pulsed mode. These advantages for inactivating organisms using intense incoherent pulsed sources include the provision of high fluences, the utilization of a wide spectral range, the spectral tunability available by varying the operating conditions and/or filtering, the high efficiency with which electrical energy is converted to light energy, the high product throughput made available by the use of intense pulsed sources, and the economics of operation.

## Claims

1. A method for aseptic packaging of foodstuffs comprising the steps of providing an aseptic packaging material having a foodstuff contacting surface intended to form at least part of the interior surface of a sealed foodstuff container, applying to said foodstuff contacting surface a plurality of high intensity polychromatic, incoherent light pulses each having a duration in the range of from 0.001 to 100 milliseconds and an energy density at said foodstuff contacting surface in the range of from 0.01 to 50 joules per square centimeter, the light pulses including ultra-violet light and light of longer wavelengths including infra-red at sufficient intensity and short pulse duration to produce pulsed thermal treatment of only a superficial surface layer of said packaging material, all to provide a sterilized foodstuff contacting surface, providing a sterilized foodstuff to be packaged, introducing the sterilized foodstuff into contact with said sterilized foodstuff contacting surface while excluding microorganisms from contact with said foodstuff and said sterilized foodstuff contacting surface, and sealing said sterilized packaging material to provide a sealed aseptically packaged foodstuff product.

2.  A method in accordance with Claim 1 wherein said light pulses have an energy distribution such that at least 10 per cent of their energy is in wavelengths shorter than 300 nanometers.

3.  A method in accordance with Claim 1 wherein said aseptic packaging material is a preformed food product container and wherein said product container is sealed by applying and sealing a sterile lid thereto.

4.  A method in accordance with Claim 1 wherein said packaging material is a flexible packaging material which is formed into a tube by longitudinally sealing said packaging material and wherein a sealed aseptically packaged foodstuff is formed by cross sealing said longitudinally sealed tube.

5.  A method in accordance with Claim 1 wherein said aseptic packaging material is contacted with an absorption enhancing agent prior to said application of said light pulses.

6.  A method in accordance with Claim 2 wherein the energy density of said light pulses at said foodstuff contacting surface is in the range of from 0.01 to 15 joules per square centimeter.

7.  A method in accordance with Claim 1 wherein said light pulses each have a duration in the range of from 0.1 to 3 milliseconds.

8.  Apparatus for performing a method of aseptic packaging of foodstuffs in accordance with Claim 1, said apparatus comprising pulsed light illumination means for applying to a foodstuff contacting surface of an aseptic packaging material a plurality of high intensity polychromatic light pulses having a duration in the range of from 0.001 millisecond to 100 milliseconds, an energy density at said foodstuff contacting surface in the range of from 0.01 joules to 50 joules per square centimeter to produce pulsed thermal treatment of only a superficial surface layer of said packaging material, means for conveying an aseptic packaging material to said pulsed light illumination means, sterilization means for providing a sterilized foodstuff to be packaged, means for introducing the sterilized foodstuff from said sterilization means into the sterilized packaging material surface provided by operation of said conveying means and said pulsed light illumination means while excluding microorganisms from contact with said foodstuff or said sterilized foodstuff contacting surface, and means for sealing said sterilized packaging material to provide a sealed foodstuff container.

**Patentansprüche**

1.  Verfahren zum aseptischen Verpacken von Lebensmitteln, umfassend die Schritte: Bereitstellen eines aseptischen Verpackungsmaterials mit einer Lebensmittel-Kontaktoberfläche, die mindestens einen Teil der Innenoberfläche eines versiegelten Lebensmittelbehälters bilden soll, Aufbringen einer Vielzahl von hochintensiven polychromatischen inkohärenten Lichtpulsen auf die LebensmittelKontaktoberfläche, von denen jeder eine Dauer im Bereich von 0,001 bis 100 Millisekunden und eine Energiedichte an der Lebensmittel-Kontaktoberfläche im Bereich von 0,01 bis 50 Joule pro Quadratzentimeter aufweist, wobei die Lichtpulse ultraviolettes Licht und Licht mit längeren Wellenlängen einschließlich Infrarot mit ausreichend Intensität und kurzer Pulsdauer enthalten, um eine Wärmepulsbehandlung von nur einer oberflächlichen Oberflächenschicht des Verpackungsmaterials zu erzeugen, alles zur Bereitstellung einer sterilisierten Lebensmittel-Kontaktoberfläche, Bereitstellen eines sterilisierten Lebensmittels, das verpackt werden soll, Inkontaktbringen des sterilisierten Lebensmittels mit der sterilisierten Lebensmittel-Kontaktoberfläche unter Ausschluß von Mikroorganismen vom Kontakt mit dem Lebensmittel und der sterilisierten Lebensmittel-Kontaktoberfläche und Versiegeln des sterilisierten Verpackungsmaterials, um ein versiegeltes, aseptisch verpacktes Lebensmittelprodukt bereitzustellen.

13

**2.** Verfahren nach Anspruch 1, worin die Lichtpulse eine derartige Energieverteilung aufweisen, daß mindestens 10 Prozent ihrer Energie in Wellenlängen von weniger als 300 Nanometer ist.

**3.** Verfahren nach Anspruch 1, worin das aseptische Verpackungsmaterial ein vorgeformter Lebensmittelbehälter ist und worin der Lebensmittelbehälter durch Anbringen und Versiegeln eines sterilen Verschlusses darauf versiegelt wird.

**4.** Verfahren nach Anspruch 1, worin das Verpackungsmaterial ein flexibles Verpackungsmaterial ist, das durch längsseitiges Versiegeln des Verpackungsmaterials zu einem Schlauch geformt wird und worin ein versiegeltes, aseptisch verpacktes Lebensmittel durch Querversiegeln des längsseitig versiegelten Schlauchs gebildet wird.

**5.** Verfahren nach Anspruch 1, worin das aseptische Verpackungsmaterial vor dem Aufbringen der Lichtpulse mit einem Absorptionsverstärkungsmittel kontaktiert wird.

**6.** Verfahren nach Anspruch 2, worin die Energiedichte der Lichtpulse an der Lebensmittel-Kontaktoberfläche im Bereich von 0,01 bis 15 Joule pro Quadratzentimeter ist.

**7.** Verfahren nach Anspruch 1, worin die Lichtpulse jeweils eine Dauer im Bereich von 0,1 bis 3 Millisekunden aufweisen.

**8.** Vorrichtung zur Durchführung eines Verfahrens zum aseptischen Verpacken von Lebensmitteln nach Anspruch 1, worin die Vorrichtung umfaßt: ein Pulslicht-Beleuchtungsmittel zum Aufbringen einer Vielzahl von hochintensiven polychromatischen Lichtpulsen auf eine Lebensmittel-Kontaktoberfläche eines aseptischen Verpackungsmaterials, die eine Dauer im Bereich von 0,001 Millisekunden bis 100 Millisekunden, eine Energiedichte an der Lebensmittel-Kontaktoberfläche im Bereich von 0,01 Joule bis 50 Joule pro Quadratzentimeter aufweisen, um eine Wärmepulsbehandlung von nur einer oberflächlichen Oberflächenschicht des Verpackungsmaterials zu bewirken, Mittel zum Befördern eines aseptischen Verpackungsmaterials zum Pulslicht-Beleuchtungsmittel, ein Sterilisierungsmittel zum Bereitstellen eines sterilisierten Lebensmittels, das verpackt werden soll, ein Mittel zum Einbringen des sterilisierten Lebensmittels aus dem Sterilisierungsmittel in die sterilisierte Verpackungsmaterialoberfläche, die durch Betrieb des Beförderungsmittels und des Pulslicht-Beleuchtungsmittels bereitgestellt ist, während Mikroorganismen vom Kontakt mit dem Lebensmittel oder der sterilisierten Lebensmittel-Kontaktoberfläche ausgeschlossen sind, und Mittel zum Versiegeln des sterilisierten Verpackungsmaterials, um einen versiegelten Lebensmittelbehälter bereitzustellen.

**Revendications**

**1.** Un procédé d'emballage aseptique de denrées alimentaires comprenant les étapes de fourniture d'un matériau d'emballage aseptique ayant une surface de contact avec une denrée alimentaire en formant au moins une partie de la surface intérieure d'un conteneur de denrée alimentaire fermé hermétiquement, d'exposition de la surface de contact avec la denrée alimentaire à des impulsions de lumière incohérente, polychromatique de haute intensité chacune ayant une durée allant de 0,001 à 100 millisecondes et une densité énergétique à la dite surface de contact avec la denrée alimentaire allant de 0,01 à 50 joules par centimètre carré, les impulsions lumineuses incluant la lumière ultraviolette et des lumières de plus grandes longueurs d'onde incluant l'infrarouge d'une intensité suffisante et d'une courte durée d'impulsion afin de réaliser un traitement thermique impulsionnel de seulement une couche superficielle de la surface du dit matériau d'emballage, le tout pour fournir une surface stérilisée destinée au contact avec la denrée alimentaire, de fourniture de denrées alimentaires stérilisées destinées à être emballées, de mise en contact des denrées alimentaires stérilisées avec la dite surface de contact avec les denrées alimentaires stérilisées, tout en écartant les microorganismes du contact des dites denrées alimentaires et de la dite surface de contact avec les denrées alimentaires stérilisées, de fermeture hermétique du dit matériau d'emballage stérilisé afin de fournir un produit alimentaire emballé et fermé hermétiquement de manière aseptique.

**2.** Un procédé selon la revendication 1 où les dites impulsions lumineuses ont une distribution énergétique telle qu'au moins 10 pour cent de leur énergie ont des longueurs d'onde inférieures à 300 nanomètres.

14

3. Un procédé selon la revendication 1 où le matériau d'emballage est un conteneur de produit alimentaire préformé et où le dit conteneur de produit est fermé hermétiquement en y présentant et y scellant un couvercle stérile.

4. Un procédé selon la revendication 1 où le dit matériau d'emballage est un matériau d'emballage souple qui est formé en un tube par le scellement longitudinal du dit matériau d'emballage et dans lequel une denrée alimentaire emballée de manière aseptique et enfermée hermétiquement est formée par le scellement transversale du dit tube scellé longitudinalement.

5. Un procédé selon la revendication 1 où le dit matériau d'emballage aseptique est en contact avec un agent améliorant l'absorption avant la dite application des dites impulsions lumineuses.

6. Un procédé selon la revendication 2 où la densité énergétique des dites impulsions lumineuses à la dite surface de contact avec la denrée alimentaire varie de 0,01 à 15 joules par centimètre carré.

7. Un procédé selon la revendication 1 où les dites impulsions lumineuses ont chacune une durée allant de 0,1 à 3 millisecondes.

8. Appareil pour la mise en oeuvre du procédé d'emballage aseptique de denrées alimentaires selon la revendication 1, le dit appareil comprenant des moyens d'éclairement en lumière impulsionnelle pour exposer une surface d'un matériau d'emballage aseptique destinée au contact avec une denrée alimentaire à une pluralité d'impulsions lumineuses polychromatiques de haute intensité d'une durée allant de 0,001 millisecondes à 100 millisecondes, d'une densité énergétique à la surface de contact avec la denrée alimentaire allant de 0,01 joules à 50 joules par centimètre carré pour réaliser un traitement thermique impulsionnel de seulement une couche superficielle de la surface du dit matériau d'emballage, des moyens pour transporter un matériau d'emballage aseptique vers les dits moyens d'éclairement en lumière impulsionnelle, des moyens de stérilisation pour fournir une denrée alimentaire stérilisée destinée à être emballée, des moyens pour présenter la denrée alimentaire stérilisée en provenance des moyens de stérilisation dans la surface du matériau d'emballage stérilisé fourni par l'opération des dits moyens de transport et des dits moyens d'éclairement en lumière impulsionnelle tout en écartant les micro-organismes du contact de la dite denrée alimentaire ou de la dite surface de contact avec la denrée alimentaire stérilisée, et des moyens de fermeture hermétique du dit matériau d'emballage stérilisé afin de fournir un conteneur de denrée alimentaire fermé hermétiquement.

FIG.1

FIG.2

FIG.3

FIG.4

FIG.5a
E. COLI
FULL SPECTRUM (+UV)

DEACTIVATION (LOGS)

ENERGY(J/cm²)

2 FLASH    4 FLASH    8 FLASH

FIG.5b
E. COLI
GLASS FILTERED SPECTRUM (-UV)

DEACTIVATION (LOGS)

ENERGY (J/cm²)

10 FLASHES    15 FLASHES    20 FLASHES

FIG. 6a
BACILLUS SUBTILIS VEGETATIVE
FULL SPECTRUM (+UV)

FIG. 6b
BACILLUS SUBTILIS VEGETATIVE
GLASS FILTERED SPECTRUM (-UV)

FIG.7a
BACILLUS SUBTILIS SPORES
FULL SPECTRUM (+UV)

FIG.7B
BACILLUS SUBTILIS SPORES
PYREX (-UV)

FIG. 8 a

STAPHYLOCOCCUS AUREUS
FULL SPECTRUM (+UV)

FIG. 8 b
STAPHYLOCOCCUS AUREUS
GLASS FILTERED SPECTRUM (-UV)

FIG.9
SACCHAROMYCES CEREVISIAE
FULL SPECTRUM (+UV)

FIG. 10a
ASPERGILLUS NIGER SPORE SUSPENSION
FULL SPECTRUM (+UV)

FIG.10 b
ASPERGILLUS NIGER SPORE SUSPENSION
GLASS FILTERED SPECTRUM (-UV)

FIG.11

SPECTRAL TRANSMISSION
(6.4 mm PYREX)

FIG.12

## FIG.13
### E. COLI DEACTIVATION IN NFDM CONTAINING MEDIA WITH U.V FILTER (-U.V) AT 4J/CM²

## FIG 14
### E.COLI DEACTIVATION IN NFDM CONTAINING MEDIA WITH U.V FILTER(-U.V)AT 4J/CM²

FIG.15

E. COLI DEACTIVATION IN INDIA INK CONTAINING MEDIA WITH UV FILTER(-UV) AT 4J/CM2